# EUROPÄISCHE PATENTSCHRIFT

(11) **EP 1 025 076 B1**
(45) Veröffentlichungstag und Bekanntmachung des Hinweises auf die Patenterteilung: **07.08.2002**
(21) Anmeldenummer: 98952728.8
(22) Anmeldetag: 21.10.1998
(51) Int. Cl.: C07C 51/44, C07C 57/04, C07C 51/48, C07C 51/25, C07C 51/50

(54) **VERFAHREN ZUR HERSTELLUNG VON (METH)ACRYLSÄURE**
METHOD FOR PRODUCING (METH)ACRYLIC ACID
PROCEDE DE PRODUCTION D'ACIDE (METH)ACRYLIQUE

(30) Priorität: 22.10.1997 DE 19746689
(43) Veröffentlichungstag der Anmeldung: 09.08.2000
(73) Patentinhaber: BASF AKTIENGESELLSCHAFT, 67056 Ludwigshafen (DE)
(72) Erfinder: HERBST, Holger, D-67227 Frankenthal (DE); NESTLER, Gerhard, A 1070 Wien (AT); HAMMON, Ulrich, D-68165 Mannheim (DE)
(74) Vertreter: Isenbruck, Günter, Dr.
(86) Internationale Anmeldenummer: EP9806678
(87) Internationale Veröffentlichungsnummer: WO9920594

(56) Entgegenhaltungen:
- EP-A- 0 717 029
- EP-A- 0 722 925
- DE-A- 3 837 955

## Beschreibung

Die Erfindung betrifft ein Verfahren zur Herstellung von (Meth)acrylsäure aus einem Gemisch, das (Meth)acrylsäure und Lösungsmittel mit einem Siedepunkt oberhalb des Siedepunktes von (Meth)acrylsäure enthält.

(Meth)acrylsäure wird als verkürzte Schreibweise verwendet und steht für Acrylsäure oder Methacrylsäure. (Meth)acrylsäure, entweder als Säure oder in Form ihrer Ester, ist insbesondere bei der Herstellung von Polymerisaten für die verschiedensten Anwendungen, z.B. als Klebstoffe, Textilhilfsmittel oder wäßrige Anstrichdispersionen, von Bedeutung.

(Meth)acrylsäure wird in der Regel durch katalytische Gasphasenoxidation von Alkanen, Alkanolen, Alkenen oder Alkenalen, die 3 bzw. 4 Kohlenstoffatome enthalten, hergestellt. Besonders vorteilhaft ist (Meth)acrylsäure z.B. durch katalytische Gasphasenoxidation von Propen. Acrolein, tert.-Butanol, iso-Buten oder Methacrolein erhältlich.

Dabei werden diese Ausgangsstoffe gasförmig, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenfalls erhöhtem Druck über Mischoxidkatalysatoren geleitet und oxidativ in die (Meth)acrylsäure umgewandelt.

Aufgrund zahlreicher im Verlauf der katalytischen Gasphasenoxidation erfolgender Parallel- und Folgereaktionen sowie aufgrund der notwendigen inerten Verdünnungsgase wird bei der katalytischen Gasphasenoxidation jedoch keine reine (Meth)acrylsäure, sondern ein Reaktionsgemisch erhalten, das im wesentlichen (Meth)acrylsäure, die inerten Verdünnungsgase und Nebenprodukte enthält. So enthält beispielsweise das bei der Oxidation von Propen anfallende Reaktionsgemisch neben Acrylsäure unter anderem Essigsäure, Propionsäure, Maleinsäureanhydrid, Aldehyde, Propan, Wasser, Kohlenoxide, Stickstoff und Sauerstoff.

Die Abtrennung der (Meth)acrylsäure aus dem Reaktionsgemisch wird im wesentlichen so durchgeführt, daß man die Reaktionsgase in einer konventionellen Absorptionskolonne zu einer absteigenden Absorptionsflüssigkeit in Gegenstrom führt, danach in einer Desorptionskolonne aus dem im wesentlichen aus (Meth)acrylsäure, dem Absorptionsmittel und Nebenkomponenten zusammengesetzten Flüssigkeitsablauf der Absorptionskolonne durch Strippen die leichtflüchtigen Nebenkomponenten weitgehend entfernt und anschließend den Flüssigkeitsablauf der Desorptionskolonne zur Abtrennung der (Meth)acrylsäure sowie schwer flüchtiger Nebenkomponenten in einer Kolonne destillativ auftrennt.

Die Abtrennung der (Meth)acrylsäure erfolgt üblicherweise, indem das Gemisch in eine einen Verstärkungsteil und einen Abtriebsteil aufweisende Kolonne eingespeist wird, wobei ein Teil der restlichen Leichtsieder, hauptsächlich Essigsäure, über den Kopf der Kolonne abgezogen wird, die (Meth)acrylsäure über einen Seitenabzug isoliert wird und im Sumpfprodukt das Absorptionsmittel und hochsiedende Nebenprodukte anfallen.

Ein derartiges Verfahren ist in DE-A 44 36 243 beschrieben. Die (Meth)acrylsäure kann auch über Kopf abgezogen werden, wie es in DE-A 43 08 087 beschrieben ist. Bei allen Aufarbeitungsschritten werden dem Produkt Stabilisatoren für die (Meth)acrylsäure, beispielsweise Phenothiazin zugesetzt. Der Zusatz der Polymerisationsinhibitoren ist notwendig, da (Meth)acrylsäure insbesondere unter thermischer Belastung eine große Neigung zur Polymerisation besitzt. Da die Polymerisate in der Regel unlöslich sind, kommt es infolge Polymerisation zur Verstopfung von Leitungen, Verdampferrohren usw. und zur Behinderung der Destillation durch Ablagerungen auf den Kolonnenböden. Aus diesem Grunde werden große Inhibitormengen eingesetzt. Aus der erhaltenen Roh-(Meth)acrylsäure kann mittels weiterer Reinigungsschritte, beispielsweise Kristallisation, eine Rein-(Meth)acrylsäure erhalten werden. Die Roh(Meth)acrylsäure weist in der Regel eine Reinheit von 80 bis 99 Gew.-%, die Rein-(Meth)acrylsäure in der Regel von mindestens 99,7 Gew.-% auf.

Bei der Weiterverarbeitung der (Meth)acrylsäure, z.B. bei der Reinigung durch Kristallisation, macht sich die große Inhibitormenge jedoch störend bemerkbar. Bei der Kristallisation wird die (Meth)acrylsäure ausgefroren, während die Verunreinigungen und die Polymerisationsinhibitoren in Lösung bleiben und zwangsläufig aufkonzentriert werden. Da manche der üblichen Inhibitoren, beispielsweise Phenothiazin, nur eine geringe Löslichkeit in (Meth)acrylsäure besitzen, ist bei tiefen Temperaturen die Löslichkeitsgrenze schnell überschritten, so daß der Polymerisationsinhibitor als Feststoff ausfällt und die ausgefrorene (Meth)acrylsäure verunreinigt.

DE-A-38 37 955 betrifft ein kontinuierliches Destillationsverfahren zur Gewinnung von beispielsweise Hydrochinon-stabilisierter Acrylsäure. Dabei wird dem Destillationsrücklauf ein Stabilisator wie Hydrochinon zugesetzt. Einigen Böden unterhalb des Rücklaufzuflusses wird ein temperaturbeständiger, stark wirksamer Inhibitor zugesetzt.

Die Aufgabe der vorliegenden Erfindung bestand darin, ein Verfahren zur destillativen Abtrennung von (Meth)acrylsäure aus einem Gemisch, das (Meth)acrylsäure und ein hochsiedendes inertes organisches Absorpcionsmittel (Lösumgsmittel) enthält, zur Verfügung zu stellen, bei dem in einer Rektifikationskolonne über einen Seitenabzug eine (Meth)acrylsäure mit geringem Polymerisationsinhibitorgehalt erhalten wird, wobei die Kolonne dabei trotzdem ausreichend stabilisiert ist, d.h. die Laufzeiten lang sind, und die gewonnene (Meth)acrylsäure eine ausreichende Lagerstabilität besitzt.

Die Aufgabe wird erfindungsgemäß gelöst durch Bereitstellung eines Verfahrens zur Herstellung von (Meth)acrylsäure aus einem Gemisch, das (Meth)acrylsäure und ein Lösungsmittel mit einem Siedepunkt oberhalb des Siedepunktes von (Meth)acrylsäure enthält, durch Destillation des Gemisches in einer Kolonne, die mit einem Verdampfer verbunden ist und einen Abtriebsteil und einen Verstärkerteil, zwischen denen das Gemisch zugeführt wird, sowie einen Seitenabzug für die (Meth)acrylsäure aufweist, wobei im Bereich des Kolonnenkopfes und unmittelbar unterhalb des Seitenabzugs ein Polymerisationsinhibitor in die Kolonne eingeführt wird, wobei die im Bereich des Kolonnenkopfes eingeführte Menge an Polymerisationsinhibitor geringer ist als die unmittelbar unterhalb des Seitenabzugs eingeführte Menge.

Vorzugsweise wird die im Bereich des Kolonnenkopfes eingeführte Menge an Polymerisationsinhibitor so gewählt, daß dieser im Rücklauf zwischen Kolonnenkopf und Seitenabzug in einer Konzentration von 5 bis 500 mg/l vorliegt, und die unmittelbar unterhalb des Seitenabzugs eingeführte Menge an Polymerisationsinhibitor so gewählt, daß dieser im Rücklauf zwischen Seitenabzug und Verdampfer in einer Konzentration von 100 bis 10000 mg/l und in der mindestens 2-fachen Konzentration im Vergleich zum Bereich zwischen Kolonnenkopf und Seitenabzug vorliegt.

Dabei werden zumindest ein Teil des Kopfproduktes im Bereich des Kolonnenkopfes und zumindest ein Teil der dem Seitenabzug entnommenen (Meth)acrylsäure unmittelbar unterhalb des Seitenabzugs nach Versetzen mit dem Polymerisationsinhibitor in die Kolonne zurückgeführt.

Unter dem Ausdruck "Lösungsmittel" ist ein hochsiedendes inertes organisches Absorptionsmittel zu verstehen, dessen Siedepunkt bei Normaldruck (1 atm) oberhalb der Siedetemperatur der (Meth)acrylsäure liegt und vorzugsweise oberhalb von 161°C liegt.

Geeignete Lösungsmittel sind beispielsweise in DE-A 21 36 396, DE-A 22 41 714 und DE-A 43 08 087 beschrieben. Beispielhaft genannt seien Mittelölfraktionen aus der Paraffindestillation, Ethylhexansäure, N-Methylpyrrolidon, Dialkylphthalate, Diphenylether, Diphenyl oder Mischungen der genannten Flüssigkeiten, z.B. Gemische aus Diphenyl, Diphenylether und gegebenenfalls o-Dimethylphthalat genannt.

Die destillative Isolierung der (Meth)acrylsäure erfolgt in einer Kolonne, die aus einem Abtriebsteil und einem Verstärkerteil mit Seitenabzug besteht, wobei sich der Seitenabzug ungefähr im mittleren Drittel, vorzugsweise knapp unterhalb des oberen Drittels des Verstärkerteils befindet. Die Rektifikationseinheit besteht vorzugsweise aus einem Abtriebsteil mit 3 bis 15 Böden und einem Verstärkerteil mit 15 bis 50 Böden. Der Zulauf für das zu trennende Gemisch befindet sich zwischen dem oberen Ende des Abtriebteils und dem unteren Ende des Verstärkerteils. Die Abtrennung der (Meth)acrytsäure erfolgt über einen Seitenabzug, der sich insbesondere im Verstärkerteil unterhalb des 5. bis 15. Bodens, vom Kolonnenkopf aus gezählt, befindet. Das Seitenabzugsprodukt wird dabei flüssig abgezogen, wobei ein Teil davon der Kolonne unterhalb des Seitenabzugs wieder zugeführt wird (unterer Rücklauf). Am unteren Ende des Abtriebsteils befinden sich ein oder mehrere Verdampfer in den dem Fachmann bekannten technischen Ausführungsmöglichkeiten. Am Kopf des Verstärkerteils werden die Brüden mit Hilfe von einem oder mehreren Kondensatoren, in den dem Fachmann bekannten technischen Ausführungsformen, niedergeschlagen. Von den niedergeschlagenen Brüden wird ein Teil als Rücklauf auf den Kopf der Kolonne zurückgeführt (oberer Rücklauf).

Als Rektifikationskolonnen kommen für das erfindungsgemäße Verfahren alle gängigen Typen in Betracht. Die Rektiftkationskolonne kann z.B. eine Boden-, Füllkörper- oder Packungskolonne sein. Vorzugsweise werden Bodenkolonnen angewendet. Beispielhaft zu nennen sind Ventil-, Glocken-, Tunnel-, Sieb- und Dualflow-Bodenkolonnen.

Die erfindungsgemäße rektifikative Abtrennung der (Meth)acrylsäure erfolgt ganz allgemein vorzugsweise bei vermindertem Druck. Es wird zweckmäßigerweise bei einem Kopfdruck < = 500 mbar, üblicherweise bei 10 bis 200 mbar, vorzugsweise bei 10 bis 100 mbar gearbeitet. In entsprechender Weise betragen die zugehörigen Temperaturen im Sumpf der Rektifikationskolonne in der Regel 100 bis 230°C, am Kopf der Kolonne 30 bis 80°C.

Die Trennlinie zwischen Verstärkerteil und Abtriebsteil der Rektifikationskolonne verläuft zweckmäßig etwa am Ende des ersten Drittels der Strecke zwischen unterster und höchstgelegener theoretischer Trennstufe. Der Seitenabzug befindet sich vorzugsweise im oberen Drittel des Verstärkerteils.

Der zur Verhinderung einer Polymerisation der (Meth)acrylsäure in der Rektifikationskolonne eingesetzte Polymerisationsinhibitor ist beispielsweise Hydrochinon, Hydrochinonmonomethylether. Paranitrosophenol, Paramethoxiphenol oder Phenothiazin oder ein Gemisch davon, vorzugsweise Phenothiazin, bevorzugt gelöst in (Meth)acrylsäure.

Die Zufuhr der Inhibitorlösung erfolgt dabei an zwei Stellen:
1. Auf dem Kolonnenkopf direkt oder vermischt mit dem oberen Rücklauf, wobei die Inhibitorkonzentration in der flüssigen Phase auf den Kolonnenböden zwischen dem Seitenabzug und dem Kopf üblicherweise 5 bis 500 mg/l, häufig 10 bis 300 mg/l und vorzugsweise 20 bis 150 mg/l beträgt. Demzufolge enthält die per Seitenabzug entnommene (Meth)acrylsäure die wie oben eingestellte Inhibitorkonzentration.
2. Unmittelbar unterhalb des Seitenabzugs direkt oder vermischt mit dem unteren Rücklauf, wobei die Inhibitorkonzentration in der flüssigen Phase auf den Kolonnenböden zwischen dem Seitenabzug und dem Sumpf üblicherweise 100 bis 10000 mg/l, häufig von 100 bis 5000 mg/l und vorzugsweise von 300 bis 1000 mg/l beträgt.

Die Wirkung des zudosierten Inhibitors kann durch Zuführung von Sauerstoff, bevorzugt in Form von Luft zum Verdampfer und/oder zur Kolonne unterstützt bzw. ergänzt werden.

Die Vorteile dieser erfindungsgemäßen Vorgehensweise sind:
- Die Inhibitorkonzentration kann in der Kolonne von oben nach unten hin variiert werden. Im oberen Kolonnenteil, wo geringere Temperaturbelastung besteht, kann mit einer geringeren Inhibitorkonzentration gearbeitet werden als im unteren Teil der Kolonne, wo die Temperarurbelastung höher ist.
   Auf diese Weise können die Inhibitorkosten reduziert werden.
- Die Inhibitorkonzentration in der Acrylsäure, die im Seitenabzug anfällt, kann dadurch so niedrig eingestellt werden, daß sie bei der Weiterverarbeitung der Säur z.B. bei der weiteren Reinigung durch Kristallisation, nicht stört.
- Es können im oberen Teil der Kolonne andere Inhibitoren eingesetzt werden als im unteren Teil der Kolonne, wo aufgrund der höheren Temperaturen unter Umständen thermostabilere Inhibitoren verwendet werden müssen.

Das Gemisch, das (Meth)acrylsäure und das Lösungsmittel enthält, kann beispielsweise erhalten werden durch
(a) Katalytische Gasphasenoxidation von Alkanen, Alkanolen, Alkenen und/oder Alkenalen mit 3 oder 4 Kohlenstoffatomen zu einem (Meth)acrylsäure enthaltenden Gasgemisch,
(b) Kontaktieren des in (a) erhaltenen Gasgemisches mit dem Lösungsmittel in einer Absorptionskolonne zur Absorption der (Meth)acrylsäure im Lösungsmittel,
(c) Strippen des in (b) erhaltenen Gemisches in einer Desorptionskolonne zur Entfernung leichtflüchtiger Nebenprodukte.

Die nähere Verfahrensweise ist eingangs beschrieben.

Nachfolgend wird die Erfindung anhand von Beispielen näher erläutert.

### Beispiel 1

Durch katalytische Gasphasenoxidation von Acrolein gemäß Beispiel B1 der DE-A 43 02 991 wurde ein Acrylsäure enthaltendes Reaktionsgemisch erzeugt. 2,1 Nm³/l dieses Reaktionsgemisches wurden in einem Gaskühler (Quench) durch Einspritzen eines Kühlmittelgemisches aus 57,4 Gew.-% Diphenylether, 20,7 Gew.-% Diphenyl und 20 Gew.-% o-Dimethylphthalat auf 170°C gekühlt.

Anschließend wurde in einem Abscheider der flüssig gebliebene Anteil des Kühlmittels von der aus Reaktionsgas und verdampftem Kühlmittel bestehenden Gasphase abgetrennt. Die eine Temperatur von 170°C aufweisende Gasphase wurde unterhalb des ersten Bodens in eine Glockenbodenkolonne mit 27 Böden geleitet und dem Gegenstrom von 3 l/h des gleichfalls aus 57,4 Gew.-% Diphenylether, 20,7 Gew.-% Diphenyl und 20 Gew.-% Dimethylphthalat zusammengesetzten, am Kolonnenkopf mit einer Temperatur von 45°C aufgegebenen Absorptionsmittels ausgesetzt. Der Ablauf der Absorptionskolonne wurde in einem Wärmetauscher indirekt auf 150°C erwärmt und auf den Kopf einer Desorptionskolonne gegeben, die als Glockenbodenkolonne mit 20 Böden ausgeführt war. In der Desorptionskolonne wurden im Vergleich zu Acrylsäure leichtsiedende Komponenten, z.B. Essigsäure und Aldehyde, durch Strippen mit Stickstoff (400 l/h, Gegenstrom) weitgehend aus dem Gemisch entfernt. Der Ablauf der Desorptionskolonne enthielt 14.7 Gew.-% Acrylsäure.

Dieser wurde mit einer Temperatur von 25°C in einer Menge von 3 l/h zwischen dem fünften und sechsten Boden (vom Verdampfer aus betrachtet) in eine 20 Glockenböden umfassende, luftdurchströmte Rektifikationskolonne geleitet. Die Rektifikationskolonne wurde mit einer Sumpftemperatur von 160°C und einem Sumpfdruck von 130°C mbar sowie einem Kopfdruck von 80 mbar betrieben.

Zwischen dem fünfzehnten und sechzehnten Boden (vom Verdampfer aus betrachtet) wurden über einen Seitenabzug pro Stunde 1400 ml Acrylsäure flüssig in einer Reinheit von 99,7 Gew.-% kontinuierlich entnommen. Das dampfförmige Kopfprodukt wurde kondensiert (600 ml/h), mit Phenothiazin (0,01 g/l) als Polymerisationsinhibitor versetzt und bis auf 50 ml/h oberhalb des obersten Glockenbodens wieder in die Rektifikationskolonne zurückgeführt.

Das ausgeschleuste Kopfprodukt (50 ml/h) wurde wieder dem Ablauf der Absorptionskolonne zugegeben.

Außerdem wurden unmittelbar unterhalb des Seitenabzuges 960 ml/h der ausgeschleusten Acrylsäure, versetzt mit 0.05 g/l Phenothiazin, zugeführt, so daß sich zwischen dem Seitenabzug und der Zulaufstelle der Kolonne eine Konzentration von ca. 500 ppm einstellte.

Die anfallende Acrylsäure enthielt 105 ppm Phenothiazin, und erst nach einer Betriebsdauer von 410 Stunden mußte der Betrieb der Rektifikationskolonne infolge Verschmutzung des Abtriebsteils und des Verdampfers abgebrochen werden. Es trat kein Polymerisat im Verstärkerteil auf.

### Vergleichsbeispiel V1

Es wurde wie in Beispiel 1 verfahren, der Rücklauf zum Kopf der Kolonne wurde jedoch mit 0,06 g/l Phenothiazin versetzt, so daß sich zwischen dem Kopf und der Zulaufstelle der Kolonne eine Konzentration von ca. 500 ppm einstellte. Unterhalb des Seitenabzugs wurde kein zusätzlicher Inhibitor zugegeben. Nach einer Betriebsdauer von 190 Stunden mußte der Betrieb der Kolonne wegen Verschmutzung des Abtriebsteils abgebrochen werden. Die isolierte Acrylsäure enthielt 550 ppm Phenothiazin.

### Vergleichsbeispiel V2

Es wurde wie im Vergleichsbeispiel 1 verfahren. Der Rücklauf zum Kopf der Kolonne wurde jedoch mit 0.01 g/l Phenothiazin versetzt, so daß sich eine Konzentration von ca. 100 mg/l einstellte.

Die entnommene Acrylsäure enthielt 110 ppm Phenothiazin, und die Laufzeit der Destillation betrug lediglich 90 Stunden.

## Patentansprüche

1. Verfahren zur Herstellung von (Meth)acrylsäure aus einem Gemisch, das (Meth)acrylsäure und ein Lösungsmittel mit einem Siedepunkt oberhalb des Siedepunktes von (Meth)acrylsäure enthalt, durch Destillation des Gemisches in einer Kolonne, die mit einem Verdampfer verbunden ist'und einen Abtriebsteil und einen Verstarkerteil, zwischen denen das Gemisch zugefuhrt wird, sowie einen Seitenabzug für die (Meth)acrylsaure aufweist, **dadurch gekennzeichnet, daß** im Bereich des Kolonnenkopfes und unmittelbar unterhalb des Seitenabzugs ein Polymerisationsinhibitor in die Kolonne eingefuhrt wird, wobei die im Bereich des Kolonnenkopfes eingeführte Menge an Polymerisationsinhibitor geringer ist als die unmittelbar unterhalb des Seitenabzugs eingerührte Menge.

2. Verfahren nach Anspruch 1, **dadurch gekennzeichnet, daß** die im Bereich des Kolonnenkopfes eingeführte Menge an Polymerisationsinhibitor so gewählt wird, daß dieser im Rücklauf zwischen Kolonnenkopf und Seitenabzug in einer Konzentration von 5 bis 500 mg/l vorliegt, und die unmittelbar unterhalb des Seitenabzugs eingeführte Menge an Polymerisationsinhibitor so gewählt wird, daß dieser im Rücklauf zwischen Seitenabzug und Verdampfer in einer Konzentration von 100 bis 10000 mg/l und in der mindestens 2-fachen Konzentration im Vergleich zum Bereich zwischen Kolonnenkopf und Seitenabzug vorliegt.

3. Verfahren nach Anspruch 1 oder 2, **dadurch gekennzeichnet, daß** zumindest ein Teil des Kopfproduktes im Bereich des Kolonnenkopfes und zumindest ein Teil der dem Seitenabzug entnommenen (Meth)acrylsäure unmittelbar unterhalb des Seitenabzugs nach Versetzen mit dem Polymerisationsinhibitor in die Kolonne zurückgeführt werden.

4. Verfahren nach einem der Ansprüche 1 bis 3, **dadurch gekennzeichnet, daß** als Polymerisationsinhibitor Hydrochinon, Hydrochinonmonomethylether, Paranitrosophenol, Paramethoxiphenol, Phenothiazin oder Gemische davon eingesetzt werden.

5. Verfahren nach einem der Ansprüche 1 bis 4, **dadurch gekennzeichnet, daß** das Lösungsmittel einen Siedepunkt höher als 161°C aufweist.

6. Verfahren nach einem der Ansprüche 1 bis 5, **dadurch gekennzeichnet, daß** dem Verdampfer und/oder der Kolonne Sauerstoff zugeführt wird.

7. Verfahren nach einem der Ansprüche 1 bis 6, **dadurch gekennzeichnet, daß** die Destillation bei vermindertem Druck durchgeführt wird.

8. Verfahren nach einem der Ansprüche 1 bis 7, **dadurch gekennzeichnet, daß** das Lösungsmittel ein Gemisch aus Diphenylether, Diphenyl und gegebenenfalls o-Dimethylphthalat ist.

9. Verfahren nach einem der Ansprüche 1 bis 8, **dadurch gekennzeichnet, daß** das Gemisch, das (Meth)acrylsäure und das Lösungsmittel enthält, erhalten wird durch
(a) katalytische Gasphasenoxidationen von Alkanen, Alkanolen, Alkenen und/oder Alkenalen mit 3 oder 4 Kohlenstoffatomen zu einem (Meth)acrylsäure enthaltenden Gasgemisch,
(b) Kontaktieren des in (a) erhaltenen Gasgemisches mit dem Lösungsmittel in einer Absorptionskolonne zur Absorption der (Meth)acrylsäure im Lösungsmittel,
(c) Strippen des in (b) erhaltenen Gemisches in einer Desorptionskolonne zur Entfernung leichtflüchtiger Nebenprodukte.

## Claims

1. A process for the preparation of (meth)acrylic acid from a mixture which contains (meth)acrylic acid and a solvent having a boiling point above the boiling point of (meth)acrylic acid by distillation of the mixture in a column which is connected to an evaporator and has a stripping section and a rectifying section, between which the mixture is fed in, and a side take-off for the (meth)acrylic acid, wherein a polymerization inhibitor is fed into the column in the region of the top of the column and immediately below the side take-off, that amount of polymerization inhibitor which is introduced in the region of the top of the column being smaller than the amount introduced immediately below the side take-off.

2. A process as claimed in claim 1, wherein the amount of polymerization inhibitor introduced in the region of the top of the column is chosen so that said polymerization inhibitor is present in a concentration of from 5 to 500 mg/l in the reflux between the top of the column and the side take-off, and the amount of polymerization inhibitor introduced immediately below the side take-off is chosen so that said polymerization inhibitor is present in a concentration of from 100 to 10,000 mg/l in the reflux between side take-off and evaporator and in at least twice the concentration compared with the region between the top of the column and the side take-off.

3. A process as claimed in claim 1 or 2, wherein at least a part of the top product is recycled to the column in the region of the top of the column and at least a part of the (meth)acrylic acid removed from the side take-off is recycled to the column immediately below the side take-off after the addition of the polymerization inhibitor.

4. A process as claimed in any of claims 1 to 3, wherein the polymerization inhibitor used is hydroquinone, hydroquinone monomethyl ether, paranitrosophenol, paramethoxyphenol, phenothiazine or a mixture thereof.

5. A process as claimed in any of claims 1 to 4, wherein the solvent has a boiling point higher than 161°C.

6. A process as claimed in any of claims 1 to 5, wherein oxygen is fed to the evaporator or to the column.

7. A process as claimed in any of claims 1 to 6, wherein the distillation is carried out at reduced pressure.

8. A process as claimed in any of claims 1 to 7, wherein the solvent is a mixture of diphenyl ether, biphenyl and, if required, o-dimethyl phthalate.

9. A process as claimed in any of claims 1 to 8, wherein the mixture which contains (meth)acrylic acid and the solvent is obtained by
(a) catalytic gas-phase oxidations of alkanes, alkanols, alkenes or alkenals of 3 or 4 carbon atoms to give a gas mixture containing (meth)-acrylic acid,
(b) bringing the gas mixture obtained in (a) into contact with the solvent in an absorption column to absorb the (meth)acrylic acid in the solvent,
(c) stripping the mixture obtained in (b) in a desorption column to remove readily volatile by-products.

## Revendications

1. Procédé de préparation d'acide (méth)acrylique à partir d'un mélange, qui contient de l'acide (méth)acrylique et un solvant ayant un point d'ébullition supérieur au point d'ébullition de l'acide (méth)-acrylique, par distillation du mélange dans une colonne, qui est reliée à un évaporateur et qui présente une partie de rectification et une partie de concentration entre lesquelles le mélange est alimenté, ainsi qu'un soutirage latéral pour l'acide (méth)acrylique, **caractérisé en ce qu'**un inhibiteur de polymérisation est introduit dans la colonne dans la zone de la tête de colonne et immédiatement en dessous du soutirage latéral, la quantité d'inhibiteur de polymérisation introduite dans la zone de la tête de colonne étant plus faible que la quantité introduite immédiatement en dessous du soutirage latéral.

2. Procédé suivant la revendication 1, **caractérisé en ce que** la quantité d'inhibiteur de polymérisation introduite dans la zone de la tête de colonne est choisie de façon que celui-ci se présente dans le reflux entre la tête de colonne et le soutirage latéral en une concentration de 5 à 500 mg/l, et **en ce que** la quantité d'inhibiteur de polymérisation introduite immédiatement en dessous du soutirage latéral est choisie de façon que celui-ci se présente dans le reflux entre le soutirage latéral et l'évaporateur en une concentration de 100 à 10.000 mg/l et en une concentration au moins doublée en comparaison de la zone entre la tête de colonne et le soutirage latéral.

3. Procédé suivant l'une des revendications 1 et 2, **caractérisé en ce qu'**au moins une partie du produit de tête est recyclée dans la zone de la tête de colonne et au moins une partie de l'acide (méth)-acrylique prélevée au soutirage latéral est recyclée immédiatement en dessous du soutirage latéral, après addition de l'inhibiteur de polymérisation dans la colonne.

4. Procédé suivant l'une des revendications 1 à 3, **caractérisé en ce que**, comme inhibiteur de polymérisation, on met en oeuvre de l'hydroquinone, de l'éther monométhylique d'hydroquinone, du paranitrosophénol, du paraméthoxyphénol, de la phénothiazine ou des mélanges de ces substances.

5. Procédé suivant l'une des revendications 1 à 4, **caractérisé en ce que** le solvant présente un point d'ébullition supérieur à 161°C.

6. Procédé suivant l'une des revendications 1 à 5, **caractérisé en ce que** de l'oxygène est amené à l'évaporateur et/ou à la colonne.

7. Procédé suivant l'une des revendications 1 à 6, **caractérisé en ce que** la distillation est effectuée sous pression réduite.

8. Procédé suivant l'une des revendications 1 à 7, **caractérisé en ce que** le solvant est un mélange d'éther diphénylique, de diphényle et éventuellement de phtalate d'o-diméthyle.

9. Procédé suivant l'une des revendications 1 à 8, **caractérisé en ce qu'**on obtient le mélange, qui contient de l'acide (méth)acrylique et le solvant,
(a) par des oxydations catalytiques en phase gazeuse d'alcanes, d'alcanols, d'alcènes et/ou d'alcénals comportant 3 ou 4 atomes de carbone, pour former un mélange gazeux contenant de l'acide (méth)acrylique,
(b) par mise en contact du mélange gazeux obtenu sous (a) avec le solvant dans une colonne d'absorption, pour l'absorption de l'acide (méth)acrylique dans le solvant,
(c) par stripage du mélange obtenu sous (b) dans une colonne de désorption en vue d'éliminer des produits secondaires aisément volatils.
